# EUROPÄISCHE PATENTSCHRIFT

(11) **EP 0 378 044 B1**
(45) Veröffentlichungstag und Bekanntmachung des Hinweises auf die Patenterteilung: **20.01.1993**
(21) Anmeldenummer: 89810827.9
(22) Anmeldetag: 02.11.1989
(51) Int. Cl.: A61F 2/36

(54) **Blattartiger Schaft für eine Femurkopfprothese**
Flat stem for a femoral head prosthesis
Tige plate pour prothèse de tête de fémur

(30) Priorität: 10.01.1989 CH 70/89
(43) Veröffentlichungstag der Anmeldung: 18.07.1990
(73) Patentinhaber: GEBRÜDER SULZER AKTIENGESELLSCHAFT, CH-8401 Winterthur (CH)
(72) Erfinder: Stühmer, Karl-Gerhart, Dr.med., D-7980 Ravensburg (DE); Koch, Rudolf, CH-8267 Berlingen (CH)

(56) Entgegenhaltungen:
- EP-A- 0 093 869
- EP-A- 0 135 755
- EP-A- 0 169 976
- EP-A- 0 222 236
- DE-A- 2 627 569
- DE-A- 3 609 119
- FR-A- 2 549 718
- FR-A- 2 573 648
- FR-A- 2 602 672
- US-A- 2 719 522
- US-A- 4 068 324

## Beschreibung

Die Erfindung betrifft einen blattartigen Geradschaft für eine Femurkopfprothese, der in seinem distalen Bereich kreiszylindrisch ausgebildet ist, wobei der distale Kreiszylinder mit Längsschlitzen versehen ist und in einen proximalen Teil mit etwas rechteckförmigen Querschnitt übergeht, wobei ferner der proximale Teil sich entlang der Schafthöhe nach medial und lateral stetig erweitert und mit parallel zu seiner Längsachse verlaufenden, scharfkantigen Rippen versehen ist, wobei weiterhin mindestens die Mehrzahl der Rippen auf einer seiner Blattseiten eine grössere Höhe hat als auf der anderen, und wobei schliesslich die Prothesenhalsachse relativ zur Längsmittelebene des Schaftes zu der nach anterior gelegenen Blattseite hin abgewinkelt ist.

Ein Verankerungsschaft der vorstehend genannten Art ist bekannt aus der EP-A-0 222 236. Bei diesem bekannten Schaft ist der proximale Bereich nieren- oder bananenähnlich gekrümmt; durch die unterschiedlichen Höhen der Rippen auf seinen Blattseiten entsteht dabei eine umhüllende Aussenform, die einer stark gekrümmten Konvexlinse ähnelt. Mit dieser bekannten Schaftform soll eine optimale Anpassung an die anatomischen Verhältnisse im proximalen Bereich des Femurknochens erreicht werden, wobei der etwa linsenförmige Bereich des spongiosen Gewebes von der Linsenform der umhüllenden Aussenform des Schaftes "gefüllt" wird.

Die der Erfindung zugrundeliegende Aufgabe besteht nun darin, diesen bekannten Schaft so zu modifizieren, dass in seinem proximalen Bereich eine elastische Vierpunktabstützung auf dem harten kortikalen Rand des Knochens erfolgt, um einen "weicheren" Uebergang der Belastungskräfte von dem Prothesenschaft auf die Kortikalis des Knochens zu erzielen.

Diese Aufgabe wird mit der Erfindung durch die Merkmale des Kennzeichnenden Teils des Anspruchs 1 gelöst.

Durch die neue Form des Schaftquerschnittes stützt sich dieser nur noch mit den beiden äusseren, elastisch verformbaren Rippen jeder Blattseite auf dem kortikalen Rand ab; darüberhinaus wird dadurch der Tatsache Rechnung getragen, dass - für eine anatomisch korrekte "Lage" des Gelenkkopfes - die lateral/medial verlaufende Mittelebene des Schaftes gegenüber der entsprechenden Ebene des Knochens nach anterior versetzt sein muss.

Um eine "Sprengung" des nach distal sich konisch verengenden kortikalen Randes des Femurs zu verhindern, ist es vorteilhaft, wenn die aus den Blattseiten herausragenden Höhen der Rippen von proximal nach distal in Stufen verringert sind.

Eine zusätzliche Sicherheit gegen unbeabsichtigte Rotationen des Schaftes lässt sich erreichen, wenn die Blattseiten etwa vom Schnittpunkt von prothesenhalsachse und lateraler Schmalseite sich in zwei Trochanterflügeln fortsetzen, die in scharfkantigen Schneiden auslaufen.

Im folgenden wird die Erfindung anhand eines Ausführungsbeispiels im Zusammenhang mit der Zeichnung näher erläutert.
Fig. 1 ist eine Seitenansicht des neuen Schaftes;
Fig. 2 ist eine Ansicht von Fig. 1 von links;
Fig. 3 schliesslich zeigt in einer Aufsicht einen für die linke Körperseite bestimmten Schaft nach Fig. 1, implantiert in einen Femurknochen.

Im distalen Bereich ist der neue Schaft 1 etwa über 1/4 bis 1/3 seiner Höhe kreiszylindrisch ausgebildet; seine Oberfläche in diesem Bereich ist glatt poliert, um seine Gleiteigenschaften zu verbessern. Vom Ende her durchsetzt eine zentrale axiale Bohrung den ganzen distalen Bereich. Zusätzlich sind in die verbleibende Wand 4 Schlitze 3 eingeschnitten. Die Bohrung und die Schlitze 3 verleihen den Segmenten der Wand 4 eine Beweglichkeit, die dem Schaft ein Nachgeben gegenüber Unebenheiten im ausgeräumten Markkanal erlauben.

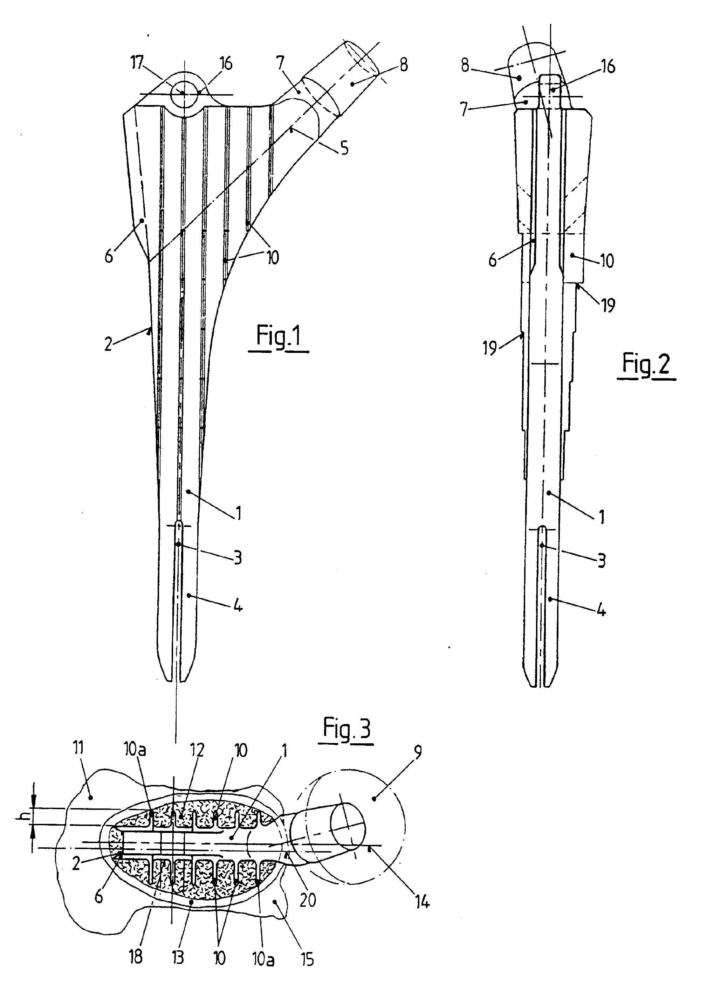

Im Anschluss an den zylindrischen Teil geht der Schaft 1 in einen Teil mit im wesentlichen rechteckigem Querschnitt über. Nach lateral und medial erweitert sich dieser Teil zunächst stetig; am Schnittpunkt der Prothesenhalsachse 5 mit der lateralen Schmalseite 2 des Schaftes 1 beginnend, sind zwei Trochanterflügel 6 an die laterale Schmalseite 2 des Schaftes 1 angesetzt, die sich bis zu seinem proximalen Ende erstrecken und als scharfe Schneidkanten ausgebildet sind. Medial verläuft der Schaft 1 in einem Bogen, der seinerseits im prothesenhals 7 endet. Dieser trägt einen konischen Zapfen 8 für den in Fig. 1 nicht gezeigten Gelenkkopf 9 (Fig. 3).

Die von den Längsseiten der Rechteckquerschnitte des Schaftkerns gebildeten Blattseiten 18 des Schaftes 1 sind mit zu seiner Längsachse parallelen Rippen 10 bedeckt, die, ebenso wie die Trochanterflügel 6, scharfkantig ausgebildet sind. Dem proximalen Abschluss des Schaftes 1 bildet eine horizontale Schulter, die von den Trochanterflügeln 6 zum prothesenhals 7 verläuft. Aus ihr ragt ein Vorsprung 16 hervor, in dem eine Bohrung 17 vorgesehen ist. Diese dient als Angriffspunkt für ein Ausziehinstrument bei eventuellen Reoperationen.

Die Blattseiten 18 des neuen Schaftes 1 sind eben ausgebildet; die Höhe h (Fig. 3) der Rippen 10 ist auf der nach anterior gerichteten Blattseite 18 geringer als auf der nach posterior gelegenen. Die "Richtungen" anterior und posterior sind aus Fig. 3 an der Lage des kleinen Trochanters 15 erkennbar, der bekanntlich leicht nach posterior gerichtet ist. Weiterhin ist die jeweilige Rippenhöhe h, die für alle Rippen einer Blattseite 18 konstant ist, auf beiden Blattseiten von proximal nach distal durch stufenförmige Absätze 19 (Fig. 2) verringert, um - wie erwähnt - die Gefahr einer "Sprengung" des Knochens 11 zu vermindern.

Wie Fig. 3 zeigt, bewirken die ebenen Blattseiten 18 und die konstante Rippenhöhe, dass sich - neben den Trochanterflügeln 6 - nur die Endrippen 10a auf der harten kortikalen Schale 13 des Knochens 11 abstützen, wodurch die geforderte elastische Vierpunkt-Abstützung des Schaftes 1 im Knochen 11 erreicht wird. Die mittleren Rippen 10 dringen jeweils nur in die Spongiosa 12 des im Querschnitt etwa linsenförmigen Knochens 11 ein.

Unterschiedliche Rippenhöhen h posterior und anterior sind angezeigt, weil, wie ebenfalls bereits geschildert, damit für eine optimale Plazierung des Gelenkkopfes 9 die Mittelebene 14 des Schaftes 1 gegenüber derjenigen 20 des Knochens 11 nach anterior versetzt angeordnet ist.

Der Prothesenhals 7 und damit der auf ihm sitzende Gelenkkopf 9 sind aus der Mittelebene 14 der Prothese um einen Winkel nach anterior herausgedreht, der einen Wert zwischen 5° und 15° annehmen kann. Durch diese zusätzliche Massnahme wird die Lage des Gelenkkopfes 9 optimal an die Stellung des natürlichen Femurkopfes angeglichen, der aufgrund der Antetorsion und der Spiralform des Femurknochens ebenfalls nach anterior "verdreht" ist.

## Patentansprüche

1. Blattartiger Geradschaft (1) für eine Femurkopfprothese, der in seinem distalen Bereich kreiszylindrisch ausgebildet ist, wobei der distale Kreiszylinder mit Längschlitzen (3) versehen ist und in einen proximalen Teil mit etwa rechteckförmigen Querschnitt übergeht, wobei ferner der proximale Teil sich entlang der Schafthöhe nach medial und lateral stetig erweitert und mit parallel zu seiner Längsachse verlaufenden, scharfkantigen Rippen (10) versehen ist, wobei weiterhin mindestens die Mehrzahl der Rippen (10) auf einer seiner Blattseiten (18) eine grössere Höhe hat als auf der anderen, und wobei schliesslich die Prothesenhalsachse (5) relativ zur Längsmittelebene (14) des Schaftes (1) zu der nach anterior gelegenen Blattseite hin abgewinkelt ist, dadurch gekennzeichnet, dass die Blattseiten (18) im proximalen Teil des Schaftes (1) eben ausgebildet sind, und dass ferner die Rippen (10) mit der geringeren Höhe (h) auf der nach anterior gelegenen Blattseite (18) angeordnet sind, wobei alle Rippen einer Blattseite im Schaftquerschnitt eine Konstante Höhe (h) und die Rippen auf verschiedenen Blattseiten im Schaftquerschnitt eine unterschiedliche Höhe (h) aufweisen.

2. Geradschaft nach Anspruch 1, dadurch gekennzeichnet, dass die aus den Blattseiten (18) herausragenden Höhen (h) der Rippen (10) von proximal nach distal in Stufen (19) verringert sind.

3. Geradschaft nach Anspruch 1 oder 2, dadurch gekennzeichnet, dass die Blattseiten (18) etwa vom Schnittpunkt von Prothesenhalsachse (5) und lateraler Schmalseite (2) sich in zwei Trochanterflügeln (6) fortsetzen, die in scharfkantigen Schneiden auslaufen.

## Claims

1. A blade-type straight stem (1) for a femur head prosthesis, the distal region of the stem forming a rirrular cylinder, in which stem the distal cylinder is provided with longitudinal slots (3) and merges into a proximal portion of substantially rectangular cross-section , in addition the proximal portion widens continuously in the medial and lateral directions along the height of the stem and is provided with sharp-edged ribs (10̸) parallel to its longitudinal axis, furthermore at least the majority of the ribs (10̸) have a greater height on one side (18) of the blade than on the other, and finally the prosthesis neck axis (5) is angled relative to the longitudinal mid plane (14) of the stem (1) towards the anterior side of the blade, characterised in that the blade sides (18) are flat in the proximal portion of the stem (1), and in addition the ribs (10̸) of lesser height (h) are situated on the anterior side (18) of the blade, the height (h) of all ribs on one blade side in the stem cross-section being constant and the height (h) of the ribs on different blade sides in the stem cross-section being different.

2. A straight stem as claimed in claim 1, characterised in that the heights (h) - projecting from the blade sides (18) - of the ribs (10̸) diminish in steps (19) from proximal to distal.

3. A straight stem as claimed in claim 1 or 2, characterised in that the blade sides (18), approximately from the point of intersection of the prosthesis neck axis (5) and the lateral narrow side (2), continue as two trochanter fins (6) which end in sharp rutting edges.

## Revendications

1. Tige droite (1) en forme de lame, pour une prothèse de tête de fémur, qui a une forme cylindrique circulaire dans sa région distale et dans laquelle le cylindre circulaire distal est pourvu de fentes longitudinales (3) et se prolonge par une partie proximale de section transversale à peu près rectangulaire, dans laquelle en outre la partie proximale s'élargit constamment le long de la hauteur de la tige, vers l'intérieur et le côté, et est pourvue de nervures (10) à arêtes vives s'étendant parallèlement à son axe longitudinal, dans laquelle en outre la majorité au moins des nervures (10) présente une plus grande hauteur sur l'une de ses faces de lame que sur l'autre, et enfin l'axe (5) du col de la prothèse est coudé par rapport au plan médian longitudinal (14) de la tige (1), vers la face de la lame située antérieurement, caractérisée en ce que les faces de lame (18) sont planes dans la partie proximale de la tige (1) et en ce qu'en outre les nervures (10) de hauteur (h) réduite sont situées sur la face de lame (18) placée antérieurement, toutes les nervures d'une face de la lame, ayant une hauteur (h) constante dans la section transversale de la tige et les nervures placées sur des côtés différents de la lame ayant une hauteur (h) différente dans la section transversale de la tige.

2. Tige droite selon la revendication 1, caractérisée en ce que les hauteurs (h) des nervures (10), dépassant des faces (18) de la lame, diminuent du côté proximal vers le coté distal, par paliers (19).

3. Tige droite selon la revendication 1 ou 2, caractérisée en ce que les faces (18) de la lame se prolongent, à partir du point d'intersection de l'axe (5) du col de prothèse et du petit côté (2) latéral, par deux ailes (6) de trochanter qui se terminent par des tranchants à arêtes vives.
